# EUROPÄISCHE PATENTSCHRIFT

(11) **EP 3 270 808 B1**
(45) Veröffentlichungstag und Bekanntmachung des Hinweises auf die Patenterteilung: **17.01.2024**
(21) Anmeldenummer: 16710972.7
(22) Anmeldetag: 18.03.2016
(51) Int. Cl.: A61B 18/14, A61B 17/11

(54) **CHIRURGISCHES GEWEBEFUSIONSINSTRUMENT**
SURGICAL TISSUE FUSION INSTRUMENT
INSTRUMENT CHIRURGICAL DE FUSION TISSULAIRE

(30) Priorität: 20.03.2015 DE 102015205057
(43) Veröffentlichungstag der Anmeldung: 24.01.2018
(73) Patentinhaber: Aesculap AG, 78532 Tuttlingen (DE)
(72) Erfinder: BARGON, Rainer, 78532 Tuttlingen (DE); BLENDER, Bernd, 78570 Mühlheim a.d. Donau (DE); EICK, Stefan, 78532 Tuttlingen (DE); HAFNER, Nikolaus, 78532 Tuttlingen (DE); HEIZMANN, Patrick, 78183 Hüfingen (DE); HERNER, Eugen, 78054 Villingen-Schwenningen (DE); HUBER, Christian, 78570 Mühlheim (DE); MERCKLE, Christof, 68199 Mannheim (DE); ODERMATT, Erich, 8200 Schaffhausen (CH); ROTHWEILER, Christoph, 78166 Donaueschingen (DE)
(74) Vertreter: Patentanwälte Ruff, Wilhelm, Beier, Dauster & Partner mbB
(86) Internationale Anmeldenummer: PCT/EP2016/055981
(87) Internationale Veröffentlichungsnummer: WO 2016/150857

(56) Entgegenhaltungen:
- DE-A1-102009 032 972
- US-A1- 2003 216 733
- US-A1- 2010 069 903
- US-A1- 2011 098 700
- US-A1- 2014 005 668

## Beschreibung

Die Erfindung betrifft ein chirurgisches Gewebefusionsinstrument mit zwei relativ zueinander beweglichen Greifstrukturen, die für das Ergreifen und Zusammenführen von biologischen Gewebeabschnitten gestaltet sind, und denen Wärmeerzeugungsmittel zugeordnet sind, die derart ausgeführt sind, dass durch einen Wärmeeintrag im Bereich der Greifstrukturen eine Gewebefusion zwischen den biologischen Gewebeabschnitten erfolgt.

Derartige chirurgische Gewebefusionsinstrumente sind als lineare oder als zirkuläre Instrumente vorgesehen. Ein lineares chirurgisches Gewebefusionsinstrument hat zwei zangenartige Greifstrukturen, die aufeinander zu beweglich sind, um zwischen sich entsprechende biologische Gewebeabschnitte zu greifen und miteinander zu verbinden. Zirkuläre chirurgische Gewebefusionsinstrumente weisen einen Basisteil mit einem zu dem Basisteil koaxial beweglichen Ambossteil auf. Die Klemmung und Verbindung erfolgt zwischen dem Ambossteil und dem Basisteil, wobei der Ambossteil über einen Ambossschaft in dem Basisteil verschiebbar gelagert ist. Die erfindungsgemäßen chirurgischen Gewebefusionsinstrumente schaffen eine Verbindung zwischen biologischen Gewebeabschnitten vorzugsweise ohne zusätzliche mechanische Verbindungsmittel wie Klammern, Fäden oder Ähnliches. Ein derartiges chirurgisches Gewebefusionsinstrument ist demzufolge insbesondere klammerlos ausgeführt. Es ist erfindungsgemäß aber auch vorgesehen, bei einem Gewebefusionsinstrument zusätzlich zu den Wärmeerzeugungsmitteln noch Vorrichtungen zur ergänzenden Einbringung mechanischer Verbindungsmittel, wie insbesondere Klammern, vorzusehen, um die Gewebefusion durch eine Klammernaht oder eine andere mechanische Verbindungsnaht zu unterstützen oder zu verstärken. Entsprechende Klammern werden vorzugsweise in einem gleichzeitigen oder vorausgehenden oder nachfolgenden Arbeitsschritt in die Gewebeabschnitte getrieben. Ein erfindungsgemäßes Gewebefusionsinstrument kann alternierend oder parallel zu entsprechenden Funktionsflächen der Wärmeerzeugungsmittel Klammerfunktionsbereiche aufweisen, denen Speicher oder Magazine zur Bevorratung der Klammern zugeordnet sind.

Aus der DE 10 2010 020 664 A1 ist ein chirurgisches Gewebefusionsinstrument bekannt, das zwei relativ zueinander bewegliche Greifstrukturen aufweist, in denen Elektroden integriert sind, die bei Strombeaufschlagung hochfrequente elektromagnetische Wellen durch die miteinander zu verbindenden biologischen Gewebeabschnitte senden, die einen Wärmeeintrag in diese Gewebeabschnitte bewirken. Dadurch erfolgt eine gewünschte Gewebefusion zwischen den biologischen Gewebeabschnitten. Zur Unterstützung und Förderung der Verbindung zwischen den biologischen Gewebeabschnitten ist eine Scheibe aus einem medizinisch verträglichen Material vorgesehen, die bei einem Zusammenführen der Greifstrukturen sandwichartig zwischen die zu verbindenden Gewebeabschnitte eingefügt wird. Zudem weist das Gewebefusionsinstrument eine Schneideinrichtung auf, die nach erfolgter Gewebefusion ein Aufteilen der miteinander fusionierten Gewebeabschnitte ermöglicht. Dies kann insbesondere bei einer A-nastomose von Hohlorganen vorteilhaft sein, um nach der Fusion der Gewebeabschnitte einen gemeinsamen Durchtrittskanal der Hohlorgane ausführen zu können.

Aus der US 2003/0216733 A1 ist ein Gewebefusionsinstrument mit einer Fluidleitung und in Elektroden integrierten Fluidaustrittsöffnungen bekannt.

Gegenstand der DE 10 2009 032 972 A1 ist eine Vorrichtung zur Herstellung von Anastomosen, wobei die Vorrichtung eine Hülse sowie einen Klebeapplikator zur Applikation eines Klebstoffs aufweist.

Die US 2014/005668 A1 betrifft ein chirurgisches Instrument mit einem Fluidmanagementsystem. Das Fluidmanagementsystem umfasst einen Fluidcontainer sowie einen Fluidkanal, der ein Fluid in Richtung eines Greiforgans des Instruments leitet.

Gegenstand der US 2010/0069903 A1 ist ein Gewebefusionsinstrument, in dessen Elektrodenbereichen ein Kanal zur Beförderung eines Fluids vorgesehen ist. Das Fluid stammt aus einem außerhalb des Instruments liegenden Reservoir. Der Austrag des Fluids in die Elektrodenbereiche erfolgt über Düsenöffnungen.

Die US 2011/0098700 A1 betrifft ein Gewebefusionsinstrument, das im Bereich seiner Elektroden ebenfalls Austrittsöffnungen zum Austritt eines Fluids besitzt.

Aufgabe der Erfindung ist es, ein chirurgisches Gewebefusionsinstrument der eingangs genannten Art zu schaffen, das eine gegenüber dem Stand der Technik weiter verbesserte Gewebefusion ermöglicht.

Diese Aufgabe wird durch den Gegenstand des unabhängigen Anspruchs 1 und die zusätzlichen Ausführungsformen in den abhängigen Ansprüchen gelöst. Insbesondere wird diese Aufgabe dadurch gelöst, dass wenigstens einer Greifstruktur ein Fluidleitungssystem zugeordnet ist, insbesondere wenigstens eine Greifstruktur ein Fluidleitungssystem aufweist, das für die Zufuhr wenigstens eines flüssigen oder fließfähigen Zusatzstoffes zu den Gewebeabschnitten während eines Gewebefusionsvorgangs ausgestaltet ist.

Der flüssige oder fließfähige Zusatzstoff unterstützt oder fördert die Fusion der biologischen Gewebeabschnitte miteinander. Der entsprechende Zusatzstoff kann eine oder mehrere der nachfolgenden Funktionen aufweisen: Förderung der Wundheilung, Erhöhung der Festigkeit der Verbindung zwischen den Gewebeabschnitten, Verbesserung einer elektrischen oder thermischen Leitfähigkeit zwischen den Wärmeerzeugungsmitteln und den Gewebeabschnitten, thermische oder elektrische Isolierung in Bereichen außerhalb des Wärmeeintrags, Vermeidung des ungewünschten Anhaftens der Gewebeabschnitte an den Greifstrukturen sowie Reduzierung der thermischen Gewebeschädigung außerhalb der Verbindungsstelle, Verbesserung des Gewebekontaktes (insbesondere zu einem Sensor oder einer Sensoranordnung des Gewebefusionsinstruments), verbesserter Energie- oder Wärmeeintrag, verbesserte Visualisierung der miteinander zu fusionierenden biologischen Gewebeabschnitte und schließlich Resorbierbarkeit des Zusatzstoffes.

Der wenigstens eine Zusatzstoff wird in flüssiger, insbesondere als Lösung, Suspension oder Emulsion, vorzugsweise als wässrige Lösung, wässrige Suspension oder wässrige Emulsion, oder fließfähiger, d.h. insbesondere partikulärer, pastöser, geschmolzener oder gelartiger, bevorzugt hydrogelartiger, Form vorgehalten, um bei einem Wärmeeintrag während eines Gewebefusionsvorgangs im Bereich der zu verbindenden Gewebeabschnitte insbesondere unter Druck- und/oder Temperatureinfluss zufließen zu können.

Die Zufuhr des wenigstens einen flüssigen oder fließfähigen Zusatzstoffs kann insbesondere durch Druckaufbau oder durch Kapillarwirkung erfolgen. Die Zufuhr des wenigstens einen Zusatzstoffs erfolgt entweder zwangsläufig mit der Aktivierung des Gewebefusionsvorgangs oder mittels einer separaten Betätigungseinrichtung je nach Bedarf.

Die erfindungsgemäße Lösung wird sowohl für zirkuläre als auch für lineare chirurgische Gewebefusionsinstrumente eingesetzt. Der Gewebefusionsvorgang umfasst das Zusammenführen der beweglichen Greifstrukturen und damit das Klemmen der biologischen Gewebeabschnitte zwischen diesen Greifstrukturen sowie den anschließenden Wärmeeintrag durch geeignete Wärmeerzeugungsmittel.

Als Wärmeerzeugungsmittel sind insbesondere Hochfrequenz- oder Radiofrequenz-Elektroden vorgesehen, die in den Greifstrukturen integriert sind. Alternativ können auch Lasereinheiten, Sonotroden, Mikrowellengeneratoren, Plasmageneratoren, Widerstandsheizgeräte wie Kauter oder eine Kombination aus zwei oder mehreren der genannten Wärmeerzeugungsmittel oder andere Wärmeerzeugungseinrichtungen als Wärmeerzeugungsmittel vorgesehen bzw. in den Greifstrukturen integriert sein. Wesentlich für alle Wärmeerzeugungsmittel, die erfindungsgemäß eingesetzt werden können, ist es, dass die Gewebeabschnitte räumlich begrenzt auf die maßgeblichen Verbindungsstellen so aufgeheizt werden, dass Strukturänderungen der Gewebeabschnitte zu der stoffschlüssigen Fusion der Gewebeabschnitte miteinander führen.

In Ausgestaltung der Erfindung weist das chirurgische Gewebefusionsinstrument einen flüssigen oder fließfähigen Zusatzstoff auf, welcher vorzugsweise in dem Fluidleitungssystem, insbesondere in wenigstens einem Fluidleitungskanal und/oder in wenigstens einem Fluidspeicher des Fluidleitungssystems, enthalten ist.

In weiterer Ausgestaltung der Erfindung ist der Zusatzstoff ausgewählt aus der Gruppe umfassend oder bestehend aus Salz wie anorganisches Salz, Wachs, Fett, Fettsäure, Alkohol, synthetisches Polymer, Biopolymer (natürlich vorkommendes Polymer), technisches Biopolymer (industriell hergestelltes Biopolymer), Protein, extrazelluläres Protein, Serumprotein, Glykoprotein, Polyaminosäure, Polyhomoaminosäure, Polyheteroaminosäure, Oligopeptid, Aminosäure, Polysaccharid, Mucopolysaccharid, Oligosaccharid, Monosaccharid, Lipid, Glykolipid, Medikament, medizinischer bzw. pharmazeutischer Wirkstoff, Wachstumsfaktor, Cyanoacrylat und Mischungen davon.

Das Salz kann ausgewählt sein aus der Gruppe umfassend oder bestehend aus Alkalimetallhalogenid, Erdalkalimetallhalogenid, Phosphat, Alkalimetallphosphat, Erdalkalimetallphosphat und Mischungen davon.

Das Salz kann insbesondere ausgewählt sein aus der Gruppe umfassend oder bestehend aus Natriumchlorid, Kaliumchlorid, Bariumchlorid, Magnesiumchlorid, Calciumchlorid, Natriumphosphat, Kaliumphosphat, Bariumphosphat, Magnesiumphosphat, Calciumphosphat, Mischphosphate davon und Mischungen davon.

Das synthetische Polymer kann beispielsweise ausgewählt sein aus der Gruppe umfassend oder bestehend aus Polyglycolid, Polylactid, Polytrimethylencarbonat, Poly-ε-Caprolacton, Poly-3-hydroxybutyrat, Poly-4-hydroxybutyrat, Poly-5-hydroxybutyrat, Poly-6-hydroxybutyrat und Mischungen davon.

Das Protein kann ausgewählt sein aus der Gruppe umfassend oder bestehend aus Kollagen, Gelatine, Elastin, Retikulin, Laminin, Fibronektin, Fibrillin, Albumin, Derivate davon, Peptidfragmente davon, Untereinheiten davon und Mischungen davon.

Bei dem Protein kann es sich insbesondere um Kollagen handeln, welches ausgewählt ist aus der Gruppe umfassend oder bestehend aus Kollagen Typ I, Kollagen Typ II, Kollagen Typ III, Kollagen Typ VI, Derivate davon, Peptidfragmente davon, Untereinheiten davon und Mischungen davon.

Das Polysaccharid kann ausgewählt sein aus der Gruppe umfassend oder bestehend aus Stärke, modifizierte Stärke, Amylose, Amylopektin, Dextran, Hyaluronsäure, Heparin, Heparansulfat, Chondroitin-4-sulfat, Chondroitin-6-sulfat, Dermatansulfat, Keratansulfat, Derivate davon und Mischungen davon.

Das Medikament kann ausgewählt sein aus der Gruppe umfassend oder bestehend aus Antibiotikum, Zytostatikum, Spasmolytikum, Thrombozytenaggregations-Hemmer, Antikoagulans, Hormon, Magen-Darm-Therapeutikum, Lokalanästhetikum, Antihypertensivum, Antiphlogistikum, Analgetikum und Mischungen davon.

Der medizinische bzw. pharmazeutische Wirkstoff kann ausgewählt sein aus der Gruppe umfassend oder bestehend aus antimikrobieller, insbesondere antibiotischer, Wirkstoff, blutstillender Wirkstoff (Hämostyptikum), entzündungshemmender Wirkstoff, wundheilungsfördernder Wirkstoff, schmerzstillender Wirkstoff, wachstumsanregender Wirkstoff und Mischungen davon.

Der antimikrobielle Wirkstoff kann ausgewählt sein aus der Gruppe umfassend oder bestehend aus Silber, Silbersalz, Antibiotikum, Polyhexamethylenbiguanid und Mischungen davon.

Der Wachstumsfaktor kann ausgewählt sein aus der Gruppe umfassend oder bestehend aus Fibroblasten-Wachstumsfaktor (FGF, Fibroblast Growth Factor), transformierender Wachstumsfaktor (TGF, Transforming Growth Factor), PDGF (Platelet-Derived Growth Factor), epidermaler Wachstumsfaktor (EGF, Epidermal Growth Factor), GMCSF (Granulocyte-Macrophage Colony Stimulating Factor), vaskulärer endothelialer Wachstumsfaktor (VEGF, Vascular Endothelial Growth Factor), insulinähnlicher Wachstumsfaktor (IGF, Insulin-like Growth Factor), Hepatozyten-Wachstumsfaktor (HGF, Hepatocyte Growth Factor), Interleukin, Nervenwachstumsfaktor (NGF, Nerve Growth Factor), hämatopoetischer Wachstumsfaktor und Mischungen davon.

In weiterer Ausgestaltung der Erfindung handelt es sich bei dem Zusatzstoff um einen gewebekontaktverbessernden Zusatzstoff. Insbesondere kann es sich bei dem Zusatzstoff um einen Zusatzstoff handeln, welcher dazu ausgebildet ist, den Gewebekontakt zu einem Sensor oder einer Sensoranordnung des chirurgischen Gewebefusionsinstruments zu verbessern. Ein derartiger Zusatzstoff kann insbesondere ein Immersionsmedium, wie beispielsweise Glycerin, Hydrogele oder Öle mit einem definierten Brechungsindex, sein.

In weiterer Ausgestaltung der Erfindung handelt es sich bei dem Zusatzstoff um einen Zusatzstoff, welcher dazu ausgebildet ist, eine bildgebende Auswertung der miteinander zu fusionierenden biologischen Gewebeabschnitte oder von bereits miteinander fusionierten biologischen Gewebeabschnitten zu verbessern. Ein geeigneter Zusatzstoff kann insbesondere als Kontaktmaterial zur besseren Einkopplung von beispielsweise Ultraschall zur bildgebenden Auswertung ausgebildet sein. Geeignete Zusatzstoffe stellen beispielsweise Hydrogele aus Carbomeren oder deren Derivaten, pflanzliche Lipogele (Oleogele), synthetische Lipogele (Oleogele), mineralische Lipogele (Oleogele), Hyaluronsäure oder biologische Gele wie Aloe Vera dar.

In weiterer Ausgestaltung der Erfindung handelt es sich bei dem Zusatzstoff um einen Zusatzstoff, welcher dazu ausgebildet ist, einen Energie- bzw. Wärmeeintrag in die miteinander zu fusionierenden biologischen Gewebeabschnitte zu verbessern. Ein geeigneter Zusatzstoff stellt/stellen beispielsweise Glycerin, biologische Öle, synthetische Öle, mineralische Öle, Hydrogele oder Lipogele (Oleogele) dar.

In weiterer Ausgestaltung der Erfindung handelt es sich bei dem Zusatzstoff um einen energiewandelnden oder -absorbierenden Zusatzstoff, welcher dazu ausgebildet ist, eine positionierte Wandlung von einer Energieform, wie beispielsweise Lichtenergie, in Wärmeenergie zu bewirken. Ein geeigneter Zusatzstoff können beispielsweise metallische oder metallisierte Partikel wie Silberpartikel, Kohlenstoffpartikel oder weitere, absorbierende Materialien oder Nanostrukturen sein. Der Zusatzstoff kann in Form von Partikeln, Emulsionen, Schmelzen oder in gelöster Form appliziert werden.

In weiterer Ausgestaltung der Erfindung handelt es sich bei dem Zusatzstoff um einen energiekoppelnden Zusatzstoff, welcher insbesondere dazu ausgebildet ist, die Einbringung von induktiver Energie zu bewirken. Bei einem solchen Zusatzstoff kann es sich beispielsweise um ferromagnetische Partikel, Graphit oder kohlenstoffhaltige Materialien handeln.

In weiterer Ausgestaltung der Erfindung handelt es sich bei dem Zusatzstoff um einen gewebsstrukturmarkierenden Zusatzstoff, welcher dazu ausgebildet ist, eine Markierung der miteinander zu fusionierenden biologischen Gewebeabschnitte oder von bereits miteinander fusionierten biologischen Gewebeabschnitten zu bewirken. Ein geeigneter Zusatzstoff stellt beispielsweise ein Fluoreszenzfarbstoff dar. Die Verwendung von Fluoreszenzfarbstoffen hat insbesondere den Vorteil, dass sich die Struktur der Gewebeabschnitte mittels einer in dem chirurgischen Gewebefusionsinstrument enthaltenen optischen Sensoranordnung detektieren lässt.

In weiterer Ausgestaltung der Erfindung handelt es sich bei dem Zusatzstoff um einen gewebsdichtemarkierenden Zusatzstoff, welcher dazu ausgebildet ist, die Dichte der miteinander zu fusionierenden biologischen Gewebeabschnitte oder von bereits miteinander fusionierten biologischen Gewebeabschnitten zu markieren, beispielsweise mittels Ultraschall. Ein geeigneter Zusatzstoff sind beispielsweise gasgefüllte Mikrobläschen ("microbubbles").

In weiterer Ausgestaltung der Erfindung handelt es sich bei dem Zusatzstoff um ein Röntgenkontrastmittel, wie beispielsweise Bariumsulfat oder ein iodhaltiges Kontrastmittel. Hierdurch ist mit besonderem Vorteil eine bildgebende Auswertung der miteinander zu fusionierenden biologischen Gewebeabschnitte oder von bereits miteinander fusionierten biologischen Gewebeabschnitten möglich.

Das Fluidleitungssystem weist wenigstens einen Fluidleitungskanal auf, der in einer der beiden Greifstrukturen integriert ist. Durch die Integration des wenigstens einen Fluidleitungskanals in eine der beiden Greifstrukturen ist eine einfache Zufuhr eines entsprechend flüssigen oder fließfähigen Fluids in Form des wenigstens einen Zusatzstoffs direkt im Bereich der Verbindungsstelle gewährleistet.

Das Fluidleitungssystem weist wenigstens einen Fluidspeicher auf, der mit dem Fluidleitungskanal verbunden ist. Der Fluidspeicher kann in dem chirurgischen Gewebefusionsinstrument integriert sein oder als separate Einheit getrennt von dem Gewebefusionsinstrument angeordnet sein. Bei einer von dem Gewebefusionsinstrument getrennten Ausführung des Fluidspeichers ist in vorteilhafter Weise ein Anschlusssystem vorgesehen, um den Fluidspeicher mit einem entsprechenden Fluidleitungskanal des Gewebefusionsinstruments verbinden zu können.

Wenigstens eine Greifstruktur ist mit Fluidaustrittsöffnungen versehen, in die der wenigstens eine Fluidleitungskanal mündet. Die Fluidaustrittsöffnungen sind vorzugsweise an der Greifstruktur so ausgerichtet, dass eine Zufuhr des flüssigen oder fließfähigen Zusatzstoffs direkt zu der Verbindungsstelle der biologischen Gewebeabschnitte bei einer Gewebefusion erfolgt.

In weiterer Ausgestaltung der Erfindung ist der wenigstens eine Fluidspeicher in wenigstens einer Greifstruktur integriert. Der flüssige oder fließfähige Zusatzstoff, der in dem Fluidspeicher gespeichert wird, muss daher lediglich kurze Wege zurücklegen, um aus dem Fluidspeicher und ggf. Fluidleitungskanälen über die Fluidaustrittsöffnungen dem Fusionsbereich der Gewebeabschnitte und damit der Verbindungsstelle zwischen den Gewebeabschnitten zugeführt werden zu können.

Der Fluidspeicher und/oder der Fluidleitungskanal weist ein abhängig von einer Betätigungseinrichtung veränderbares Speichervolumen auf. Das veränderbare Speichervolumen ermöglicht druckabhängig einen Austrag des flüssigen und fließfähigen Zusatzstoffs über die Fluidaustrittsöffnungen in Richtung der zu verbindenden Gewebeabschnitte.

Die Betätigungseinrichtung ist mit einer Betätigungseinheit zum Zusammenführen der Greifstrukturen in Wirkverbindung. Eine Betätigung der Greifstrukturen in Richtung ihrer Schließstellung führt demzufolge zwangsläufig auch zu einem Fluidaustrag des Zusatzstoffs im Bereich der Fluidaustrittsöffnungen. Eine zusätzliche, separate Betätigung ist nicht erforderlich, um den Zusatzstoff ausbringen zu können.

In weiterer Ausgestaltung der Erfindung sind der Fluidspeicher und/oder die Betätigungseinrichtung getrennt von dem Gewebefusionsinstrument positioniert, und es sind lösbare Anschlussmittel vorgesehen, um eine Verbindung zwischen dem Fluidspeicher und/oder der Betätigungseinrichtung und dem wenigstens einen Fluidleitungskanal des Gewebefusionsinstruments herzustellen oder zu lösen. Die Anschlussmittel sind vorzugsweise mechanische Verbindungssysteme in Form von Schlauchkupplungen, Luer-Lock- oder Luer-Slip-Verbindungen oder Ähnlichem.

Die Fluidaustrittsöffnungen sind in wenigstens einer Elektrodenanordnung integriert, die den Wärmeerzeugungsmitteln zugeordnet ist. Vorteilhaft ist die Elektrodenanordnung als Hochfrequenz-Elektrodenanordnung (HF-Elektrode) oder als Radiofrequenz-Elektrodenanordnung ausgeführt.

Die Fluidaustrittsöffnungen sind in einer beweglich gelagerten Betätigungswandung des wenigstens einen Fluidleitungskanals oder Fluidspeichers vorgesehen, die abhängig von einer Aktivierung der Betätigungseinheit zum Zusammenführen der Greifstrukturen verlagerbar ist. Durch die Verlagerung erfolgt druckabhängig der gewünschte Fluidaustritt des Zusatzstoffs in Richtung der Verbindungsstelle der Gewebeabschnitte. Vorteilhaft ist die Betätigungswandung schwimmend in einem Strukturkörper der Greifstruktur gelagert. Die Betätigungswandung ist durch eine Elektrodenwandung der Elektrodenanordnung gebildet.

Vorzugsweise sind die Fluidaustrittsöffnungen in einem als Greifstruktur dienenden, linearbeweglichen Ambossteil eines zirkulären Gewebefusionsinstruments integriert. In vorteilhafter Weise umfasst der Ambossteil wenigstens einen Fluidspeicher und wenigstens einen Fluidleitungskanal. Vorteilhaft ist die Betätigungswandung in dem Ambossteil integriert. Hierdurch kann in einfacher Weise eine direkte Zufuhr des wenigstens einen Zusatzstoffs zu dem Fusionsbereich der biologischen Gewebeabschnitte zwischen den Greifstrukturen, d.h. zwischen dem Ambossteil und einem Basisteil des zirkulären Gewebefusionsinstruments, erfolgen. In vorteilhafter Weise weist der Ambossteil einen Ambosskopf und einen Ambossschaft auf, der in dem Basisteil längsverschiebbar gelagert ist. Der Basisteil weist vorteilhaft entsprechende Wärmeeintragmittel, vorzugsweise in Form einer Elektrodenanordnung, auf. Der Ambosskopf kann ergänzend mit komplementären Wärmeerzeugungsmitteln, insbesondere in Form einer zu der Elektrodenanordnung im Basisteil abgestimmten Elektrodenanordnung, versehen sein. Vorzugsweise sind im Ambossteil und im Basisteil miteinander zusammenwirkende Elektrodenanordnungen als Wärmeerzeugungsmittel vorgesehen. Alternativ kann entweder der Ambossteil oder der Basisteil mit einem Wärmeerzeugungsmittel versehen sein.

Vorteilhaft ist eine Betätigungswandung des Fluidspeichers als Betätigungskolben der Betätigungseinrichtung ausgeführt, der abhängig von einer Verlagerungsbewegung des Ambossteils relativ zum Basisteil bewegt wird. Eine Betätigung der Greifstrukturen des zirkulären Gewebefusionsinstrumentes bewirkt demzufolge zwangsläufig einen Austrag des Zusatzstoffs aus den Fluidaustrittsöffnungen auf die Verbindungsstelle zwischen den biologischen Gewebeabschnitten.

In weiterer Ausgestaltung der Erfindung ist das Fluidleitungssystem in einem wenigstens einen Teilbereich des Gewebefusionsinstruments ummantelnden Trägergehäuse integriert, das mit dem wenigstens einen Fluidleitungskanal oder dem Fluidspeicher sowie mit den Fluidaustrittsöffnungen versehen ist. Das Trägergehäuse kann in vorteilhafter Weise separat zu dem Gewebefusionsinstrument hergestellt und nach der Fertigstellung und Befüllung mit dem Zusatzstoff mit dem Gewebefusionsinstrument verbunden werden. Das Trägergehäuse wird auf entsprechende Außenkonturen des Gewebefusionsinstrumentes je nach linearer oder zirkulärer Ausführung des Gewebefusionsinstrumentes abgestimmt.

In weiterer Ausgestaltung der Erfindung sind die Fluidaustrittsöffnungen in dem Trägergehäuse im Bereich der Trennebene zwischen den Greifstrukturen positioniert, und die Fluidaustrittsöffnungen sind radial von außen auf die Trennebene gerichtet. Die Positionierung der Fluidaustrittsöffnungen ist auf die auf dem Gewebefusionsinstrument montierte Position des Trägergehäuses bezogen. Die radiale Anordnung der Fluidaustrittsöffnungen ermöglicht einen Fluidaustrag des Zusatzstoffs von außen auf die Verbindungsstelle der Gewebeabschnitte.

In weiterer Ausgestaltung der Erfindung ist ein zirkuläres Gewebefusionsinstrument vorgesehen, und das Trägergehäuse ist als einen Basisteil des zirkulären Gewebefusionsinstruments hülsenförmig oder rohrförmig umschließender Hohlprofilkörper gestaltet, der mit einem über seine gesamte Länge durchgängigen Aufweitungsschlitz versehen ist, um den Hohlprofilkörper auf dem Basisteil zu montieren oder von diesem zu lösen. Der Aufweitungsschlitz ermöglicht ein Aufklipsen oder Entfernen des Hohlprofilkörpers relativ zum Basisteil. Vorzugsweise erfolgt die Montage oder Demontage des Hohlprofilkörpers relativ zum Basisteil werkzeuglos. Der Hohlprofilkörper umfasst die Fluidaustrittsöffnungen, die in montiertem Zustand vorteilhaft in Richtung der Verbindungsstelle zwischen den Greifstrukturen ragen. Zudem umfasst der Hohlprofilkörper wenigstens einen Fluidleitungskanal. Der Hohlprofilkörper kann mit einem Anschlussstutzen zur Verbindung mit einem Fluidspeicher versehen sein. Alternativ kann der Hohlprofilkörper selbst auch den Fluidspeicher umfassen. In vorteilhafter Weise ist der Hohlprofilkörper einstückig ausgeführt und die Fluidaustrittsöffnungen, der wenigstens eine Fluidleitungskanal sowie gegebenenfalls der Fluidspeicher sind einstückig in diesem Hohlprofilkörper integriert.

In weiterer Ausgestaltung der Erfindung ist der Hohlprofilkörper mit Anschlussmitteln für einen Fluidspeicher und/oder eine Betätigungseinrichtung zur Förderung eines flüssigen Zusatzstoffs in Richtung der Fluidaustrittsöffnungen versehen. Als Fluidspeicher kann eine Spritze oder ein anders gestaltetes Behältnis vorgesehen sein. Als Anschlussmittel sind vorzugsweise Luer-Verbindungsmittel in Form von Luer-Lock- oder Luer-Slip-Verbindungsprofilierungen vorgesehen.

In weiterer Ausgestaltung der Erfindung weist das chirurgische Gewebefusionsinstrument einen Sensor oder eine Sensoranordnung auf. Bei dem Sensor bzw. der Sensoranordnung kann es sich um einen elektronischen Sensor bzw. eine elektronische Sensoranordnung, Temperatursensor bzw. Temperatursensoranordnung oder um einen optischen Sensor, insbesondere spektroskopischen Sensor, bzw. eine optische Sensoranordnung, insbesondere spektroskopische Sensoranordnung, handeln. Vorzugsweise ist der Sensor bzw. die Sensoranordnung in einem Handgriff des chirurgischen Gewebefusionsinstruments integriert.

In weiterer Ausgestaltung der Erfindung weist das chirurgische Gewebefusionsinstrument eine Energiequelle, insbesondere einen Stromgenerator, vorzugsweise Hochfrequenzstromgenerator, einen Radiofrequenzgenerator, einen Ultraschallwellengenerator, einen Mikrowellengenerator oder eine Lichtquelle, insbesondere einen Laser, auf. Vorzugsweise ist die Energiequelle in einem Handgriff des chirurgischen Gewebefusionsinstruments integriert.

In weiterer Ausgestaltung der Erfindung weist das chirurgische Gewebefusionsinstrument ein Energieübertragungselement auf, welches dazu ausgebildet ist, einen Energieübertrag von einer Energiequelle des chirurgischen Gewebefusionsinstruments auf wenigstens eine der beiden Greifstrukturen, insbesondere auf beide Greifstrukturen, bevorzugt auf das Wärmeerzeugungsmittel/die Wärmeerzeugungsmittel, zu bewirken. Bei dem Übertragungselement kann es sich beispielsweise um elektrische Leitungen oder Lichtleiter, d.h. transparente Bauteile, wie Fasern, Röhren oder Stäbe, welche Licht über kurze oder lange Strecken transportieren können, handeln.

In weiterer Ausgestaltung der Erfindung weist das chirurgische Gewebefusionsinstrument einen Akkumulator (Akku) auf. Der Akkumulator ist dazu ausgebildet, das Gewebefusionsinstrument, insbesondere eine darin integrierte Energiequelle, mit Strom zu versorgen. Bei dem Akkumulator kann es sich prinzipiell um eine Akkumulatorzelle (Akkuzelle) oder um einen Akkupack handeln. Der Akkumulator ist vorzugsweise (ebenfalls) in einem Handgriff des chirurgischen Gewebefusionsinstruments integriert.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt eine Ausführungsform eines erfindungsgemäßen chirurgischen Gewebefusionsinstruments in linearer Ausführung,
- Fig. 2: in vergrößerter Darstellung eine Greifstruktur des Gewebefusionsinstruments nach Fig. 1,
- Fig. 3: in verkleinerter Darstellung die Greifstruktur nach Fig. 2 mit einem angeschlossenen Fluidspeicher,
- Fig. 4: in einer Schnittdarstellung einen Ausschnitt der Greifstruktur nach Fig. 2 im Bereich eines Fluidleitungskanals und mehrerer Fluidaustrittsöffnungen,
- Fig. 5 und 6: weitere Schnittdarstellungen durch den Zangenteil, aus denen die Anordnungen von Fluidleitungskanälen und Fluidaustrittsöffnungen erkennbar sind,
- Fig. 7: eine weitere Ausführungsform eines erfindungsgemäßen chirurgischen Gewebefusionsinstruments in zirkulärer Ausführung,
- Fig. 8: in vergrößerter Darstellung einen Längsschnitt durch einen Teilbereich des Gewebefusionsinstruments nach Fig. 7 vor einer Zusammenführung von Basisteil und Ambossteil,
- Fig. 9: die Ausführungsform nach Fig. 8 mit zusammengefügtem Ambossteil und Basisteil,
- Fig. 10: Teilbereiche des Gewebefusionsinstruments nach den Fig. 7 bis 9 mit einem Ambossteil vor einer Verbindung mit einem Trokardorn,
- Fig. 11: die Darstellung nach Fig. 10, jedoch nach der Verbindung des Ambossteils mit dem Trokardorn,
- Fig. 12: eine weitere Ausführung eines Ambossteils ähnlich den Fig. 10 und 11 für den Einsatz bei einem Gewebefusionsinstrument gemäß den Fig. 8 und 9,
- Fig. 13: den Ambossteil nach Fig. 12 in betätigter Stellung seines Fluidspeichers,
- Fig. 14: in einer Explosionsdarstellung eine weitere Ausführungsform eines erfindungsgemäßen Gewebefusionsinstruments ähnlich Fig. 7,
- Fig. 15: das Gewebefusionsinstrument nach Fig. 14 in montiertem Zustand,
- Fig. 16: in einer Schnittdarstellung einen Teilbereich einer weiteren Ausführungsform eines erfindungsgemäßen Gewebefusionsinstruments ähnlich den Fig. 7 bis 15 mit kombinierten Fluidleitungseinheiten,
- Fig. 17: einen als Trägergehäuse für das Fluidleitungssystem dienenden Hohlprofilkörper gemäß Fig. 14 und 15 und
- Fig. 18: einen Querschnitt durch den Hohlprofilkörper nach Fig. 17 entlang der Schnittlinie XVIII-XVIII.

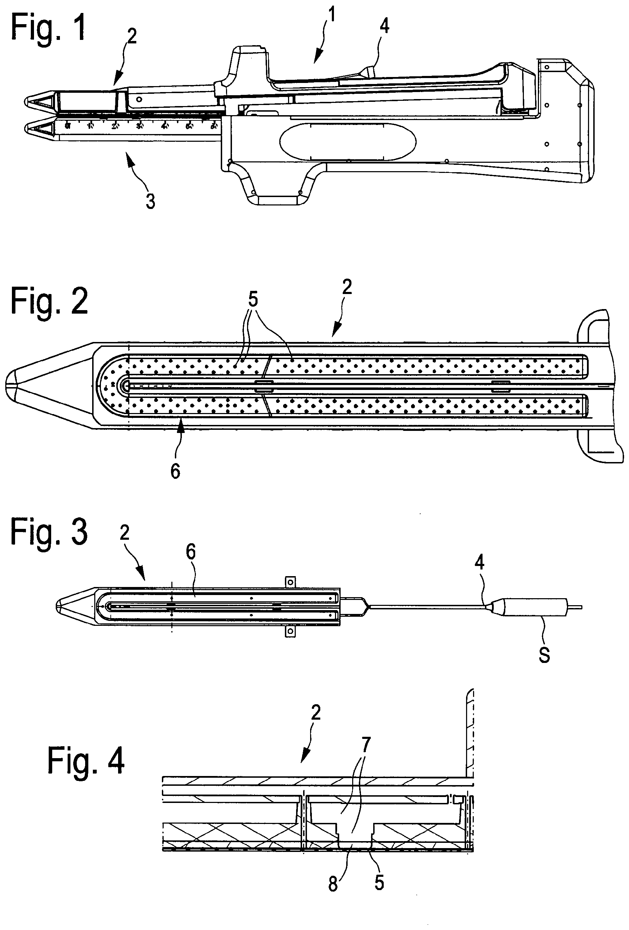

Ein chirurgisches Gewebefusionsinstrument 1 gemäß den Fig. 1 bis 6 ist ein lineares Gewebefusionsinstrument. Das lineare Gewebefusionsinstrument 1 gemäß den Fig. 1 bis 6 weist zwei relativ zueinander bewegliche Greifstrukturen 2, 3 auf, von denen eine untere Greifstruktur 3 stationär, d.h. ortsfest, relativ zu einem nicht näher bezeichneten Basisteil des Gewebefusionsinstruments 1 gehalten ist. Die in Fig. 1 obere Greifstruktur 2 ist an einem relativ zu dem Basisteil hubbeweglichen Oberteil angeordnet, so dass die beiden Greifstrukturen 2, 3 in Hochrichtung relativ zueinander linear aufeinander zu- oder voneinander wegbewegt werden können. Die Greifstrukturen 2, 3 bewirken daher eine lineare Klemmung der entsprechenden biologischen Gewebeabschnitte, sobald das Gewebefusionsinstrument 1 durch Zusammenführen der Greifstrukturen 2, 3 geschlossen ist. Zwischen den Greifstrukturen 2, 3 werden biologische Gewebeabschnitte längs einer linearen Verbindungsstelle miteinander verbunden. Jede Greifstruktur 2, 3 weist als Wärmeerzeugungsmittel jeweils eine Elektrodenanordnung 6 auf, die elektrisch mit Strom beaufschlagbar sind, um einen Wärmeeintrag in die biologischen Gewebeabschnitte im Bereich der Verbindungsstelle einzubringen. Der Wärmeeintrag über die Elektrodenanordnungen 6 bewirkt die gewünschte Gewebefusion. Die Elektrodenanordnungen 6 sind als Hochfrequenz-Elektroden ausgeführt. Die Greifstrukturen 2, 3 können zusätzlich noch ein nicht näher bezeichnetes Schneidwerkzeug umfassen, um überschüssiges Gewebematerial abtrennen zu können. Jede Elektrodenanordnung 6 in den Greifstrukturen 2, 3 weist eine Elektrodenoberfläche auf, die in zueinander parallelen Ebenen flächig längserstreckt sind. Jede Elektrodenoberfläche ist Teil einer Kontaktfläche jeder Greifstruktur 2, 3, die beim Schließen des Gewebefusionsinstrumentes die Gewebeabschnitte kontaktieren und zwischen sich einklemmen. Bei der Ausführung gemäß Fig. 2 und 3 sind die Elektrodenoberflächen nach Art einer Schleife U-förmig verlegt. Entsprechende Elektrodenoberflächen der einander zugewandten Elektrodenanordnungen 6 der beiden Greifstrukturen 2, 3 sind zusätzlich mit einer Vielzahl von Fluidaustrittsöffnungen 5 versehen, um bei einem Gewebefusionsvorgang direkt im Bereich der Verbindungsstelle zwischen den zu verbindenden biologischen Gewebeabschnitten einen flüssigen oder fließfähigen Zusatzstoff aufbringen zu können. Die Fluidaustrittsöffnungen 5 stellen Mündungsbereiche von Fluidleitungskanälen oder Fluidspeichern 7, 8 dar, die in der jeweiligen Greifstruktur 2, 3 integriert sind.

Es ist erfindungsgemäß auch möglich, lediglich eine der beiden Greifstrukturen 2, 3 mit Fluidleitungskanälen 7, 8 und mit Fluidaustrittsöffnungen 5 für den wenigstens einen flüssigen oder fließfähigen Zusatzstoff zu versehen, so dass nur in der einen Greifstruktur 2 ein entsprechender Zusatzstoff geführt ist. Bei dieser Ausführung wird der Zusatzstoff während eines Gewebefusionsvorgangs lediglich von einer Seite auf die Verbindungsstelle zwischen den biologischen Gewebeabschnitten aufgebracht.

Die Fluidleitungskanäle 7, 8 bilden zusätzlich ein Fluidreservoir für den wenigstens einen flüssigen oder fließfähigen Zusatzstoff. Damit bilden zumindest Teile der Fluidleitungskanäle 7, 8 auch einen Fluidspeicher für den Zusatzstoff im Sinne der Erfindung. Die Fluidleitungskanäle 7, 8 sind über Anschlussmittel in Form eines Anschlussstutzens 4 zudem für eine bedarfsabhängige Nachförderung des Zusatzstoffs aus einer ebenfalls als Fluidspeicher dienenden Spritze S ausgelegt. Der Anschlussstutzen 4 ist über eine Schlauch- oder Rohrleitung, die als Fluidleitungskanal dient, mit dem zuvor beschriebenen Fluidreservoir verbunden. In wenigstens einer Greifstruktur 2 ist demzufolge ein Fluidleitungssystem aus Fluidleitungskanälen 7, 8, Fluidaustrittsöffnungen 5 sowie wenigstens einem Fluidspeicher integriert.

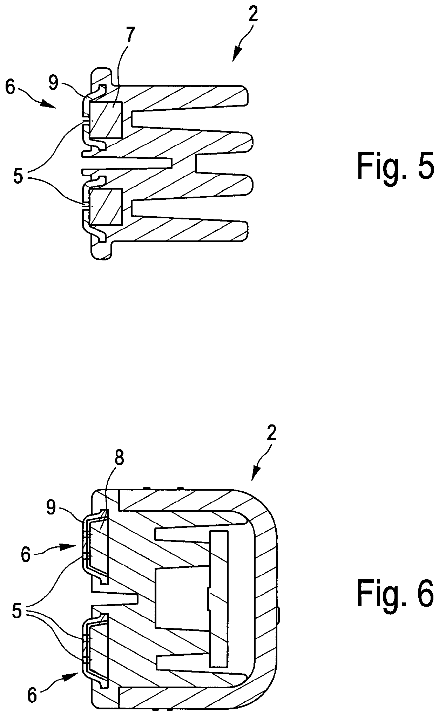

Anhand der Fig. 5 und 6, die verschiedene Querschnitte längs der Greifstruktur 2 zeigen, sind die unterschiedlichen Tiefen der Fluidleitungskanäle 7, 8 und demzufolge die unterschiedlichen Volumina der Fluidleitungskanäle 7, 8 erkennbar. Zudem ist erkennbar, dass die Elektrodenoberfläche der Elektrodenanordnung 6 durch eine streifen- oder blattförmige Elektrodenwandung 9 gebildet ist, die schwimmend in einem formstabilen Strukturkörper der Greifstruktur 2 gelagert ist. Der Strukturkörper bildet im Bereich seiner Oberfläche einen Teil der Kontaktfläche der Greifstruktur 2. Die Elektrodenwandung 9 ist thermisch und/oder elektrisch leitend ausgeführt. In die Elektrodenwandung 9 sind die Fluidaustrittsöffnungen 5 eingebracht.

Anhand der Fig. 5 und 6 ist erkennbar, dass die streifen- oder blattförmigen Elektrodenwandungen 9 der Elektrodenanordnungen 6 das jeweilige, in der Greifstruktur ausgebildete Fluidreservoir, d.h. die Fluidleitungskanäle oder Fluidspeicher 7 und 8, zur Verbindungsstelle hin verschließen. Die Elektrodenwandungen 9 sind schwimmend gelagert oder alternativ elastisch flexibel ausgeführt. Sobald das Gewebefusionsinstrument 1 durch Zusammenführen der Greifstrukturen 2, 3 geschlossen wird, werden zwangsläufig die Elektrodenwandungen 9 verlagert oder deformiert, wodurch sich in den Fluidreservoiren ein erhöhter Druck aufbaut, der dazu führt, dass der flüssige oder fließfähige Zusatzstoff aus den Fluidaustrittsöffnungen 5 in Richtung zur Verbindungsstelle gedrückt, d.h. gefördert, wird.

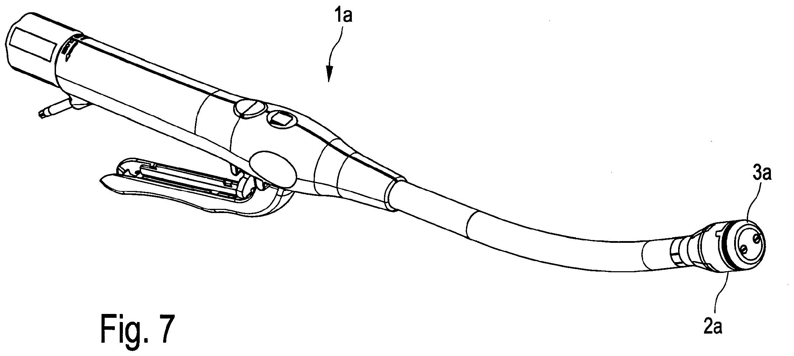

Ein chirurgisches Gewebefusionsinstrument 1a gemäß den Fig. 7 bis 11 ist wie das zuvor beschriebene Gewebefusionsinstrument 1 dazu vorgesehen, biologische Gewebeabschnitte insbesondere klammerlos durch Zusammenführung, Klemmung und Wärmeeintrag miteinander zu verbinden, d.h. zu fusionieren. Das chirurgische Gewebefusionsinstrument 1a gemäß den Fig. 7 bis 11 ist als zirkuläres Gewebefusionsinstrument gestaltet, das biologische Gewebeabschnitte in Form von Hohlorganen miteinander verbinden kann. Auch bei dem zirkulären Gewebefusionsinstrument erfolgt ein thermischer Energieeintrag an einer zirkulären, d.h. kreisförmigen Verbindungsstelle über kreisförmig angeordnete Elektrodenanordnungen 6a, die als Hochfrequenz-Elektroden ausgeführt sind. Auch das zirkuläre Gewebefusionsinstrument 1a weist zwei Greifstrukturen 2a, 3a auf, die relativ zueinander beweglich angeordnet sind, um gegeneinandergepresst oder auseinanderbewegt zu werden. Die eine Greifstruktur 3a ist relativ zu der anderen Greifstruktur 2a linear beweglich gelagert, wie nachfolgend näher beschrieben wird. Die linear bewegliche Greifstruktur 3a wird auch als Ambossteil bezeichnet, wohingegen die relativ zum Gewebefusionsinstrument 1a ortsfest verbleibende, gegenüberliegende Greifstruktur 2a als Basisteil bezeichnet wird. Dem Gewebefusionsinstrument 1a ist eine nicht bezeichnete Energieversorgungsleitung zugeführt, die, insbesondere als Stromkabel, das über ein Stromnetz im Betrieb des Gewebefusionsinstruments 1a elektrische Energie zuführt, die über die Elektrodenanordnungen 6a den gewünschten Wärmeeintrag in die Verbindungsstelle zwischen den Greifstrukturen 2a, 3a einbringt. Alternativ kann das Gewebefusionsinstrument 1a mittels eines Akkumulators mit Strom versorgt werden. Die Verwendung eines Akkumulators erlaubt mit besonderem Vorteil den Einbau einer Energiequelle, insbesondere eines Hochfrequenzstromgenerators, in das chirurgische Gewebefusionsinstrument 1a. Bevorzugt sind in diesem Fall die Energiequelle sowie der Akkumulator in einem Handgriff des Gewebefusionsinstruments 1a integriert.

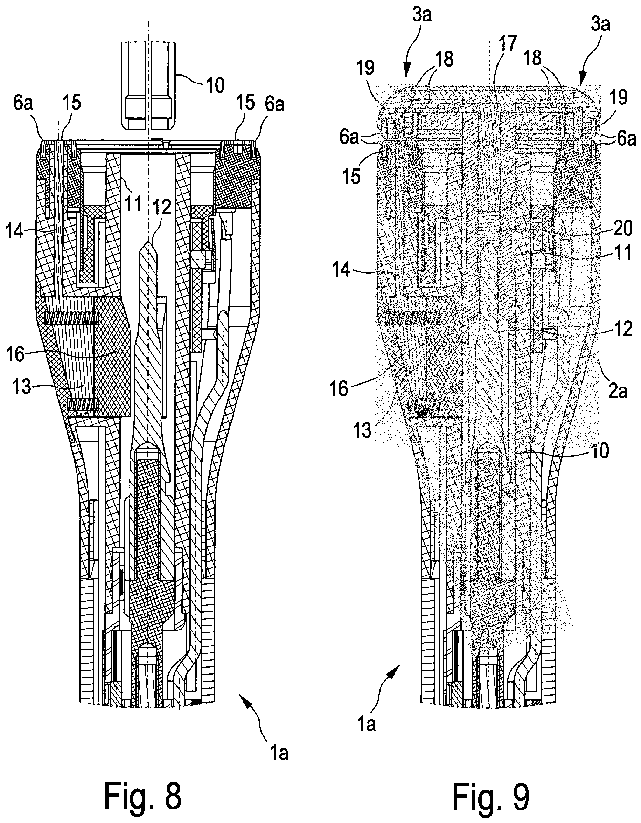

Der Ambossteil 3a weist einen Ambossschaft 10 auf, der auf einen in dem Basisteil 2a koaxial zu einer Mittellängsachse längsverschiebbar gelagerten Trokardorn 12 koaxial aufsteckbar und auf diesem verrastbar ist (Fig. 8 und 9).

Anhand der Fig. 8 und 9 ist erkennbar, dass in dem Basisteil 2a radial innerhalb der Elektrodenanordnung 6a eine ringförmige Schneideinheit positioniert ist, die Teil einer in dem Gewebefusionsinstrument 1a integrierten Schneideinrichtung ist. Die Schneideinrichtung kann mechanisch ausgeführt sein und wenigstens eine Schneidklinge umfassen, oder auch nichtmechanisch, insbesondere in Form einer Laserschneideinrichtung. Die nicht näher bezeichnete ringförmige Schneidklinge kann bei Bedarf in eine Verbindungsebene zwischen dem Ambossteil 3a und dem Basisteil 2a hineinbewegt werden, um so ein kreisförmiges Aufteilen der miteinander verbundenen Gewebeabschnitte zu bewirken.

Dem Basisteil 2a des Gewebefusionsinstruments 1a ist ein Fluidspeicher 13 zugeordnet, der in einem Aufnahmeabschnitt des Basisteils 2a integriert ist. Der Fluidspeicher 13 ist zur Speicherung eines flüssigen oder fließfähigen Zusatzstoffs vorgesehen und steht über einen oder mehrere Fluidleitungskanäle 14 mit Fluidaustrittsöffnungen 15 im Bereich der ringförmigen Elektrodenanordnung 6a in Verbindung. Die Fluidaustrittsöffnungen 15 sind in entsprechenden Elektrodenoberflächen der Elektrodenanordnung 6a integriert. Die Elektrodenoberflächen sind durch wenigstens eine streifen- oder blattförmige Elektrodenleiste gebildet, die in einer Stirnseite des Basisteils 2a gehalten ist (Fig. 8 und 9). Die Elektrodenleiste wird auch als Elektrodenwandung bezeichnet. Kontaktflächen der Greifstrukturen 2a, 3a sind analog der Ausführung des linearen Gewebefusionsinstrumentes durch Elektrodenanordnungen und benachbarte Oberflächen der die Elektrodenanordnungen tragenden Strukturkörper der Greifstrukturen 2a, 3a gebildet.

Der Fluidspeicher 13 in dem Basisteil 2a ist durch eine Betätigungseinrichtung beaufschlagbar, durch die ein Volumen des Fluidspeichers 13 komprimierbar ist. Der entsprechende Druckaufbau führt zwangsläufig zu einem Austrag des Zusatzstoffs durch die Fluidaustrittsöffnungen 15. Der Fluidspeicher 13 weist eine radial nach innen in einen Führungskanal 11 des Basisteils 2a ragende Betätigungstaste 16 auf, die über eine Federanordnung in Form von Schraubendruckfedern an einer Außenwandung des Fluidspeichers 13 abgestützt ist. Die Betätigungstaste 16 ist radial zu dem Führungskanal 11 linear beweglich gelagert. Der Ambossschaft 10 weist vorliegend einen Außenmantel mit axial verlaufenden Aussparungen auf, die mit der Betätigungstaste 16 derart zusammenwirken, dass ein Außenmantel des Ambossschafts 10 oberhalb der axialen Aussparungen mit der Betätigungstaste 16 in Kontakt gerät, sobald der Ambossteil 3a in seine Schließstellung verfahren ist. Dadurch wird die Betätigungstaste 16 radial nach außen gedrückt und führt zu dem Druckaufbau innerhalb des Fluidspeichers 13, der den gewünschten Zusatzstoffaustrag im Bereich der Fluidaustrittsöffnungen 15 des Basisteils 2a bewirkt.

Auch in dem Ambossteil 3a ist ein Fluidleitungssystem zur Ausbringung eines flüssigen oder fließfähigen Zusatzstoffs integriert. Das Fluidleitungssystem im Ambossteil 3a umfasst einen Fluidspeicher 17, Fluidleitungskanäle 18 sowie Fluidaustrittsöffnungen 19. Der Fluidspeicher 17 ist in dem Ambossschaft 10 ausgebildet. Hierzu ist der Ambossschaft hohl gestaltet. Der Fluidspeicher 17 ist auf einer dem Trokardorn 12 zugewandten Seite mittels eines als Betätigungswandung dienenden Verschlusskolbens 20 verschlossen. Sobald der Ambossteil 3a in die Schließstellung des Gewebefusionsinstruments geführt wird, wodurch ein Ambosskopf des Ambossteils 3a gegen eine Stirnfläche der Elektrodenanordnung 6a des Basisteils 2a gedrückt wird, bewegt sich der Ambossschaft 10 relativ zu dem Trokardorn 12. Dies bewirkt zwangsläufig eine Bewegung des Verschlusskolbens 20 längs des Ambossschafts 10 in Richtung des Ambosskopfs, wodurch das Volumen des Fluidspeichers 17 komprimiert wird. Dies führt zwangsläufig zu einem Fluidaustritt des Zusatzstoffs im Bereich der Fluidaustrittsöffnungen 19.

Beim Schließen des zirkulären Gewebefusionsinstruments 1a erfolgt somit gleichzeitig von gegenüberliegenden Seiten her eine Zufuhr des flüssigen oder fließfähigen Zusatzstoffs im Bereich der Verbindungsstelle sowohl aus dem Basisteil 2a als auch aus dem Ambossteil 3a heraus.

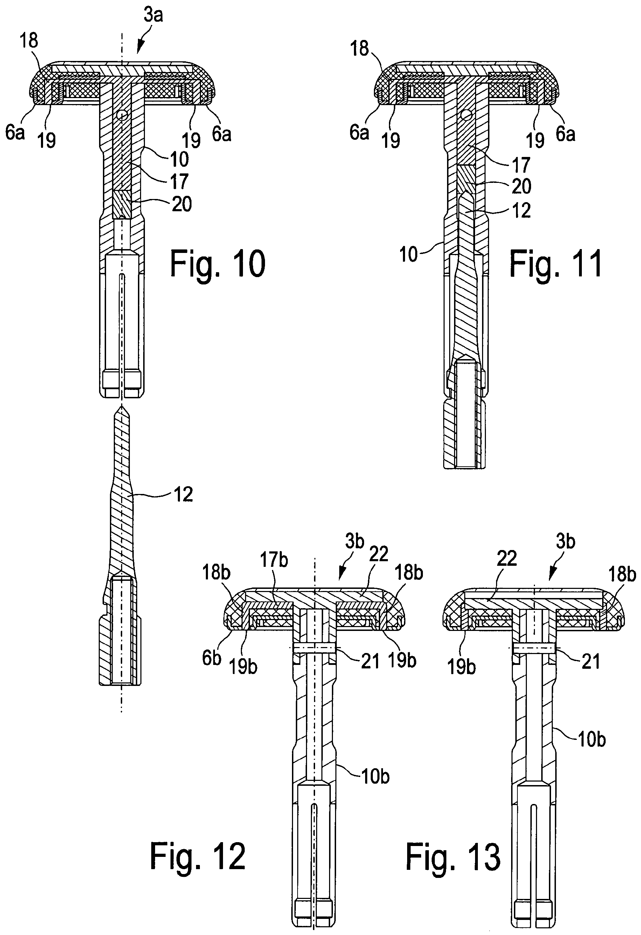

Alternativ zu dem Ambossteil 3a nach den Fig. 10 und 11 kann das zirkuläre Gewebefusionsinstrument 1a nach den Fig. 7 bis 9 auch mit einem Ambossteil 3b gemäß den Fig. 12 und 13 betrieben werden. Dieser Ambossteil 3b unterscheidet sich von dem Ambossteil 3a dadurch, dass der Ambossschaft 10b relativ zu dem Ambosskopf koaxial verschiebbar angeordnet ist. An dem Ambossschaft 10b ist hierzu stirnseitig eine Kopfplatte 22 angeordnet, die eine obere Betätigungswandung für einen in dem Ambosskopf angeordneten Fluidspeicher 17b bildet. Eine Bewegung des Ambossschafts 10b relativ zu dem Ambosskopf bewirkt eine Volumenreduzierung des Fluidspeichers 17b, so dass über Fluidleitungskanäle 18b ein Zusatzstoffaustrag im Bereich der Fluidaustrittsöffnungen 19b erfolgt. Um einen entsprechenden Druckaufbau zu erzielen, wird der Ambossteil 3b in seine Schließstellung relativ zu dem Basisteil verfahren. Sobald der Ambosskopf im Bereich der Verbindungsstelle an der Stirnfläche des Basisteils zur Anlage kommt, ist der Ambosskopf gegen eine weitere Längsbewegung blockiert. Der nicht dargestellte Trokardorn hingegen bewegt den Ambossschaft 10b in gleicher Richtung weiter, wodurch der Ambossschaft 10b über einen formschlüssig wirksamen Verbindungsbolzen 21 die als Kolben dienende Deckplatte 22 mitnimmt, so dass eine gewünschte Volumenreduzierung im Fluidspeicher 17b auftritt.

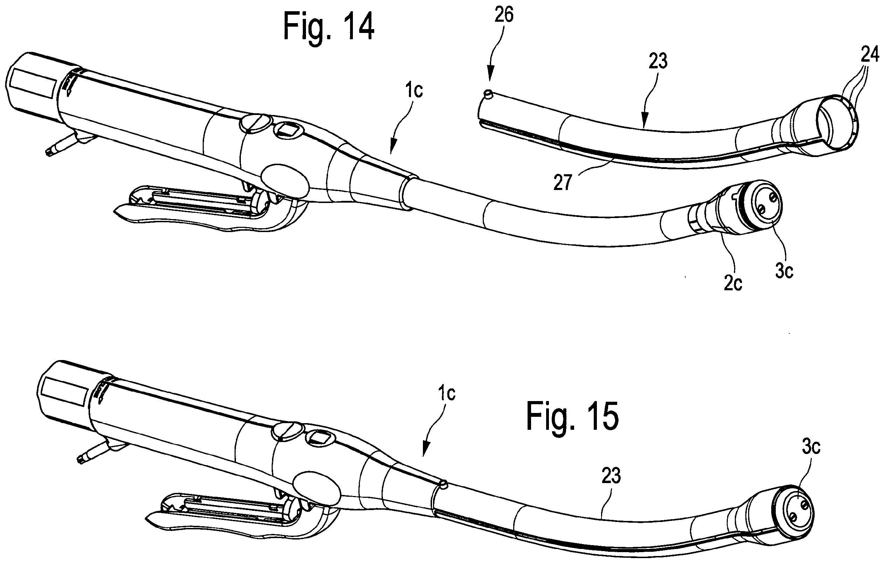

Bei der Ausführungsform nach den Fig. 14 und 15 sowie 17 und 18 ist dem zirkulären Gewebefusionsinstrument 1c ebenfalls ein Fluidleitungssystem zugeordnet, um einen flüssigen oder fließfähigen Zusatzstoff im Bereich der Verbindungsstelle zwischen Ambossteil 3c und Basisteil 2b bei Bedarf zuzuführen. Die Zufuhr des Zusatzstoffs bei dieser Ausführungsform erfolgt jedoch nicht axial wie bei den zuvor beschriebenen Ausführungsformen, sondern radial von au-ßen. Hierzu ist ein Trägergehäuse 23 in Form eines Hohlprofilkörpers vorgesehen, der über seine Länge mittels eines Aufweitungsschlitzes 27 durchgängig geschlitzt ist. Der Hohlprofilkörper ist hülsen- oder rohrförmig gestaltet und ist doppelwandig ausgeführt, um in dem Doppelwandungsbereich mehrere über den Umfang des Hohlprofilkörpers verteilt angeordnete Fluidleitungskanäle 25 auszubilden. Die Fluidleitungskanäle sind ringförmig über den Hohlprofilkörper verteilt und erstrecken sich über eine gesamte Länge des Hohlprofilkörpers zwischen radial nach innen gerichteten Fluidaustrittsöffnungen 24 im Bereich einer Stirnseite des Hohlprofilkörpers und einem als Anschlussmittel dienenden Eintrittsstutzen 26 an einem gegenüberliegenden Stirnendbereich des Hohlprofilkörpers. Der Eintrittsstutzen 26 dient dazu, einen Fluidspeicher, in dem der Zusatzstoff gespeichert ist, anzuschließen. Alternativ kann der Zusatzstoff auch direkt in den Fluidleitungskanälen 25 gespeichert sein. Bei dieser Ausführung dient der Eintrittsstutzen als Anschlussmittel für eine Betätigungseinrichtung, um in den Fluidleitungskanälen 25 Druck aufbauen zu können und demzufolge einen Austrag des Zusatzstoffs im Bereich der Fluidaustrittsöffnungen 24 zu ermöglichen.

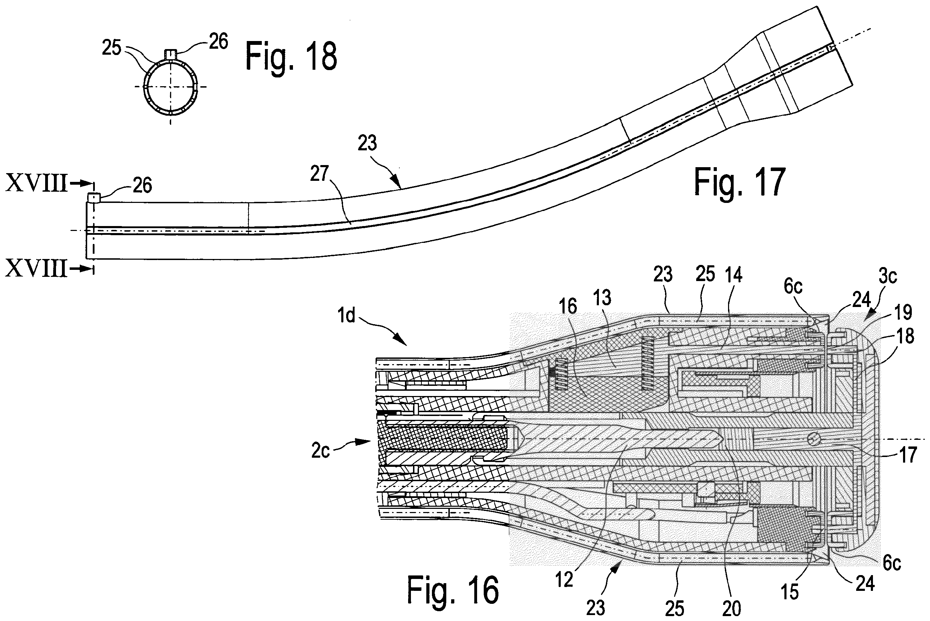

Anhand der Fig. 16 ist ein weiteres zirkuläres Gewebefusionsinstrument 1d ähnlich den zuvor beschriebenen Ausführungsformen dargestellt. Wesentlicher Unterschied bei dem Gewebefusionsinstrument 1d ist es, dass dort alle anhand der zuvor beschriebenen Ausführungsformen gemäß den Fig. 7 bis 15, 17 und 18 vorhandenen Fluidleitungssysteme miteinander kombiniert sind. Dies bedeutet, dass der Basisteil 2c zum einen von einem Trägergehäuse 23 gemäß den Fig. 14 und 15, 17 und 18 umschlossen ist. Zum anderen sind die Fluidleitungssysteme im Basisteil 2c und im Ambossteil 3c vorgesehen, wie sie anhand der Fig. 8 bis 11 zuvor bereits beschrieben worden waren.

## Patentansprüche

1. Chirurgisches Gewebefusionsinstrument (1, 1a, 1b, 1c) mit zwei relativ zueinander beweglichen Greifstrukturen (2, 3; 2a, 3a; 2b, 3b; 2c, 3c), die für das Ergreifen und Zusammenführen von biologischen Gewebeabschnitten gestaltet sind, und denen Wärmeerzeugungsmittel zugeordnet sind, die derart ausgeführt sind, dass durch einen Wärmeeintrag im Bereich der Greifstrukturen eine Gewebefusion zwischen den biologischen Gewebeabschnitten erfolgt, wobei wenigstens einer Greifstruktur (2, 3; 2a, 3a; 2b, 3b; 2c, 3c) ein Fluidleitungssystem zugeordnet ist, das für die Zufuhr wenigstens eines flüssigen oder fließfähigen Zusatzstoffs zu den Gewebeabschnitten während eines Gewebefusionsvorgangs ausgestaltet ist, wobei das Fluidleitungssystem wenigstens einen Fluidleitungskanal (7, 8; 14; 18, 18b) aufweist, der in einer der beiden Greifstrukturen integriert ist und wenigstens eine Greifstruktur (2, 3; 2a, 3a; 2b, 3b) mit Fluidaustrittsöffnungen (5, 15, 19; 19b) versehen ist, in die der wenigstens eine Fluidleitungskanal (7, 8; 14; 18; 18b) mündet, wobei das Fluidleitungssystem wenigstens einen Fluidspeicher (7, 8; 13; 17; 17b) aufweist, der mit dem Fluidleitungskanal (7, 8; 14; 18; 18b) verbunden ist, wobei der Fluidspeicher (13, 17, 17b) und/oder der Fluidleitungskanal (7, 8, 14, 18, 18b) ein abhängig von einer Betätigungseinrichtung veränderbares Speichervolumen aufweist, wobei die Betätigungseinrichtung mit einer Betätigungseinheit zum Zusammenführen der Greifstrukturen (2, 3; 2a, 3a; 2b, 3b) in Wirkverbindung ist, wobei die Fluidaustrittsöffnungen (5, 15, 19, 19b) in wenigstens einer Elektrodenanordnung (6, 6a, 6b) integriert sind, die den Wärmeerzeugungsmitteln zugeordnet ist, wobei die Fluidaustrittsöffnungen (5) in einer beweglich gelagerten Betätigungswandung (9) des wenigstens einen Fluidleitungskanals (7, 8) oder Fluidspeichers vorgesehen sind, die abhängig von einer Aktivierung der Betätigungseinheit zum Zusammenführen der Greifstrukturen verlagerbar ist,
**dadurch gekennzeichnet, dass** die Betätigungswandung (9) durch eine Elektrodenwandung der Elektrodenanordnung (6, 6a, 6b) gebildet ist.

2. Chirurgisches Gewebefusionsinstrument (1, 1a, 1b, 1c) nach Anspruch 1, **dadurch gekennzeichnet, dass** der wenigstens eine Fluidspeicher (7, 8; 13; 17; 17b) in wenigstens einer Greifstruktur (2, 2a; 3a; 2b, 3b) integriert ist.

3. Chirurgisches Gewebefusionsinstrument (1, 1a, 1b, 1c) nach Anspruch 1 oder 2, **dadurch gekennzeichnet, dass** der Fluidspeicher und/oder die Betätigungseinrichtung getrennt von dem Gewebefusionsinstrument ( 1c) positioniert sind, und dass Anschlussmittel (4, 26) vorgesehen sind, um eine Verbindung zwischen dem Fluidspeicher und/oder der Betätigungseinrichtung und dem wenigstens einen Fluidleitungskanal (7, 8; 25) des Gewebefusionsinstruments (1, 1c) herzustellen oder zu lösen.

4. Chirurgisches Gewebefusionsinstrument (1, 1a, 1b, 1c) nach einem der vorhergehenden Ansprüche, **dadurch gekennzeichnet, dass** das Fluidleitungssystem in einem wenigstens einen Teilbereich des Gewebefusionsinstruments (1c) ummantelnden Trägergehäuse (23) integriert ist, das mit dem wenigstens einen Fluidleitungskanal (25) oder dem Fluidspeicher sowie mit den Fluidaustrittsöffnungen (24) versehen ist.

5. Chirurgisches Gewebefusionsinstrument (1, 1a, 1b, 1c) nach Anspruch 4, **dadurch gekennzeichnet, dass** die Fluidaustrittsöffnungen (24) in dem Trägergehäuse (23) im Bereich einer Trennebene zwischen den Greifstrukturen (2c, 3c) positioniert sind, und dass die Fluidaustrittsöffnungen (24) radial von außen auf die Trennebene gerichtet sind.

6. Chirurgisches Gewebefusionsinstrument (1, 1a, 1b, 1c) nach Anspruch 4 oder 5, **dadurch gekennzeichnet, dass** ein zirkuläres Gewebefusionsinstrument vorgesehen ist, und dass das Trägergehäuse (23) als einen Basisteil (2c) des zirkulären Gewebefusionsinstruments (1c) hülsenförmig oder rohrförmig umschließender Hohlprofilkörper gestaltet ist, der mit einem über seine gesamte Länge durchgängigen Aufweitungsschlitz (27) versehen ist, um den Hohlprofilkörper auf dem Basisteil zu montieren oder von diesem zu lösen.

7. Chirurgisches Gewebefusionsinstrument (1, 1a, 1b, 1c) nach Anspruch 6, **dadurch gekennzeichnet, dass** der Hohlprofilkörper (23) mit Anschlussmitteln (26) für einen Fluidspeicher und/oder eine Betätigungseinrichtung zur Förderung eines flüssigen oder fließfähigen Zusatzstoffes in Richtung der Fluidaustrittsöffnungen (24) versehen ist.

## Claims

1. Surgical tissue fusion instrument (1, 1a, 1b, 1c) with two gripping structures (2, 3; 2a, 3a; 2b, 3b; 2c, 3c) which are movable relative to each other, are designed for gripping and bringing together biological tissue sections, and are assigned heat-generating means designed in such a way that tissue fusion takes place between the biological tissue sections by heat being supplied in the area of the gripping structures, wherein at least one gripping structure (2, 3; 2a, 3a; 2b, 3b; 2c, 3c) is assigned a fluid-conducting system, which is designed to supply at least one liquid or flowable additive to the tissue sections during a tissue fusion procedure, wherein the fluid-conducting system has at least one fluid-conducting channel (7, 8; 14; 18, 18b), which is integrated in one of the two gripping structures and at least one gripping structure (2, 3; 2a, 3a; 2b, 3b) is provided with fluid outlet openings (5, 15, 19; 19b), into which the at least one fluid-conducting channel (7, 8; 14; 18; 18b) opens, wherein the fluid-conducting system has at least one fluid reservoir (7, 8; 13; 17; 17b), which is connected to the fluid-conducting channel (7, 8; 14; 18; 18b), wherein the fluid reservoir (13, 17, 17b) and/or the fluid-conducting channel (7, 8, 14, 18, 18b) has a reservoir volume that is variable depending on an actuation mechanism, wherein the actuation mechanism is operatively connected to an actuation unit for bringing together the gripping structures (2, 3; 2a, 3a; 2b, 3b), wherein the fluid outlet openings (5, 15, 19, 19b) are integrated in at least one electrode arrangement (6, 6a, 6b), which is assigned to the heat-generating means, wherein the fluid outlet openings (5) are provided in a movably mounted actuation wall (9) of the at least one fluid-conducting channel (7, 8) or fluid reservoir, which actuation wall (9), depending on an activation of the actuation unit, is movable in order to bring together the gripping structures, **characterized in that** the actuation wall (9) is formed by an electrode wall of the electrode arrangement (6, 6a, 6b).

2. Surgical tissue fusion instrument (1, 1a, 1b, 1c) according to Claim 1, **characterized in that** the at least one fluid reservoir (7, 8; 13; 17; 17b) is integrated in at least one gripping structure (2, 2a; 3a; 2b, 3b).

3. Surgical tissue fusion instrument (1, 1a, 1b, 1c) according to Claim 1 or 2, **characterized in that** the fluid reservoir and/or the actuation mechanism are positioned separate from the tissue fusion instrument (1c), and **in that** attachment means (4, 26) are provided for producing or cancelling a connection between the fluid reservoir and/or the actuation mechanism and the at least one fluid-conducting channel (7, 8; 25) of the tissue fusion instrument (1, 1c).

4. Surgical tissue fusion instrument (1, 1a, 1b, 1c) according to one of the preceding claims, **characterized in that** the fluid-conducting system is integrated in a carrier housing (23) which encloses at least a partial area of the tissue fusion instrument (1c) and which is provided with the at least one fluid-conducting channel (25) or the fluid reservoir and also with the fluid outlet openings (24).

5. Surgical tissue fusion instrument (1, 1a, 1b, 1c) according to Claim 4, **characterized in that** the fluid outlet openings (24) in the carrier housing (23) are positioned in the area of a dividing plane between the gripping structures (2c, 3c), and **in that** the fluid outlet openings (24) are directed radially from the outside towards the dividing plane.

6. Surgical tissue fusion instrument (1, 1a, 1b, 1c) according to Claim 4 or 5, **characterized in that** a circular tissue fusion instrument is provided, and **in that** the carrier housing (23) is designed as a hollow profile body which forms a sleeve-shaped or tubular enclosure around a base part (2c) of the circular tissue fusion instrument (1c) and which is provided with an expansion slit (27) extending along its entire length, so as to be able to mount the hollow profile body on the base part or detach it therefrom.

7. Surgical tissue fusion instrument (1, 1a, 1b, 1c) according to Claim 6, **characterized in that** the hollow profile body (23) is provided with attachment means (26) for a fluid reservoir and/or an actuation mechanism for conveying a liquid or flowable additive in the direction of the fluid outlet openings (24).

## Revendications

1. Instrument chirurgical de fusion tissulaire (1, 1a, 1b, 1c) avec deux structures de préhension (2, 3 ; 2a, 3a ; 2b, 3b ; 2c, 3c) mobiles l'une par rapport à l'autre, qui sont conçues pour saisir et réunir des sections de tissu biologique, et auxquelles sont associés des moyens de production de chaleur qui sont réalisés de telle sorte que, par un apport de chaleur dans la zone des structures de préhension, il se produit une fusion tissulaire entre les sections de tissu biologique, à au moins une structure de préhension (2, 3 ; 2a, 3a ; 2b, 3b ; 2c, 3c) étant associé un système de conduite de fluide qui est conçu pour l'amenée d'au moins un additif liquide ou fluide aux sections de tissu pendant une opération de fusion tissulaire, le système de conduite de fluide présentant au moins un canal de conduite de fluide (7, 8 ; 14 ; 18, 18b) qui est intégré dans l'une des deux structures de préhension et au moins une structure de préhension (2, 3 ; 2a, 3a ; 2b, 3b) étant pourvue d'ouvertures de sortie de fluide (5, 15, 19 ; 19b) dans lesquelles l'au moins un canal de conduite de fluide (7, 8 ; 14 ; 18, 18b) débouche, le système de conduite de fluide présentant au moins un réservoir de fluide (7, 8 ; 13 ; 17 ; 17b) qui est relié au canal de conduite de fluide (7, 8 ; 14 ; 18 ; 18b), le réservoir de fluide (13, 17, 17b) et/ou le canal de conduite de fluide (7, 8, 14, 18, 18b) présentant un volume de stockage modifiable en fonction d'un dispositif d'actionnement, le dispositif d'actionnement étant en liaison fonctionnelle avec une unité d'actionnement pour réunir les structures de préhension (2, 3 ; 2a, 3a ; 2b, 3b), les ouvertures de sortie de fluide (5, 15, 19, 19b) étant intégrées dans au moins un agencement d'électrode (6, 6a, 6b) qui est associé aux moyens de production de chaleur, les ouvertures de sortie de fluide (5) étant prévues dans une paroi d'actionnement (9) montée mobile de l'au moins un canal de conduite de fluide (7, 8) ou réservoir de fluide, qui peut être déplacée en fonction d'une activation de l'unité d'actionnement pour réunir les structures de préhension, **caractérisé en ce que** la paroi d'actionnement (9) est formée par une paroi d'électrode de l'agencement d'électrode (6, 6a, 6b).

2. Instrument chirurgical de fusion tissulaire (1, 1a, 1b, 1c) selon la revendication 1, **caractérisé en ce que** l'au moins un réservoir de fluide (7, 8 ; 13 ; 17 ; 17b) est intégré dans au moins une structure de préhension (2, 2a ; 3a ; 2b, 3b).

3. Instrument chirurgical de fusion tissulaire (1, 1a, 1b, 1c) selon la revendication 1 ou 2, **caractérisé en ce que** le réservoir de fluide et/ou le dispositif d'actionnement sont positionnés séparément de l'instrument de fusion tissulaire (1c), et **en ce que** des moyens de raccordement (4, 26) sont prévus afin d'établir ou de défaire une liaison entre le réservoir de fluide et/ou le dispositif d'actionnement et l'au moins un canal de conduite de fluide (7, 8 ; 25) de l'instrument de fusion tissulaire (1, 1c).

4. Instrument chirurgical de fusion tissulaire (1, 1a, 1b, 1c) selon l'une quelconque des revendications précédentes, **caractérisé en ce que** le système de conduite de fluide est intégré dans un boîtier de support (23) enveloppant au moins une zone partielle de l'instrument de fusion tissulaire (1c), qui est pourvu de l'au moins un canal de conduite de fluide (25) ou du réservoir de fluide ainsi que des ouvertures de sortie de fluide (24).

5. Instrument chirurgical de fusion tissulaire (1, 1a, 1b, 1c) selon la revendication 4, **caractérisé en ce que** les ouvertures de sortie de fluide (24) sont positionnées dans le boîtier de support (23) dans la zone d'un plan de séparation entre les structures de préhension (2c, 3c), et **en ce que** les ouvertures de sortie de fluide (24) sont dirigés radialement de l'extérieur vers le plan de séparation.

6. Instrument chirurgical de fusion tissulaire (1, 1a, 1b, 1c) selon la revendication 4 ou 5, **caractérisé en ce qu'**un instrument circulaire de fusion tissulaire est prévu, et **en ce que** le boîtier de support (23) est conçu sous forme de corps profilé creux entourant en forme de manchon ou en forme de tube une partie de base (2c) de l'instrument circulaire de fusion tissulaire (1c), qui est pourvu d'une fente d'élargissement (27) continue sur toute sa longueur, afin de monter le corps profilé creux sur la partie de base ou de le défaire de celle-ci.

7. Instrument chirurgical de fusion tissulaire (1, 1a, 1b, 1c) selon la revendication 6, **caractérisé en ce que** le corps profilé creux (23) est pourvu de moyens de raccordement (26) pour un réservoir de fluide et/ou un dispositif d'actionnement pour le transport d'un additif liquide ou fluide en direction des ouvertures de sortie de fluide (24).
